(19) Europäisches Patentamt
European Patent Office
Office européen des brevets

(11) Veröffentlichungsnummer: **0 138 139**
**A2**

(12) # EUROPÄISCHE PATENTANMELDUNG

(21) Anmeldenummer: **84111736.9**

(22) Anmeldetag: **02.10.84**

(51) Int. Cl.[4]: **C 07 C 143/70**, C 07 C 155/02

(30) Priorität: **14.10.83 JP 190861/83**

(43) Veröffentlichungstag der Anmeldung: **24.04.85**
**Patentblatt 85/17**

(84) Benannte Vertragsstaaten: **BE CH DE FR GB IT LI**

(71) Anmelder: **NIHON TOKUSHU NOYAKU SEIZO K.K.,**
**No.4, 2-chome, Nihonbashi Honcho Chuo-ku,**
**Tokyo 103 (JP)**

(72) Erfinder: **Saito, Junichi, 3-7-12, Osawa, Mitaka-shi Tokyo (JP)**
Erfinder: **Takemoto, Toshiyuki, 167-6, Inume-cho, Hachioji-shi Tokyo (JP)**

(74) Vertreter: **Schumacher, Günter, Dr. et al, c/o Bayer AG**
**Konzernverwaltung RP Patentabteilung,**
**D-5090 Leverkusen 1 Bayerwerk (DE)**

(54) **Neues Verfahren zur Herstellung substituierter Benzolsulfonylchloride.**

(57) Die Erfindung betrifft ein neues Verfahren zur Herstellung eines substituierten Benzolsulfonylchlorids der allgemeinen Formel I

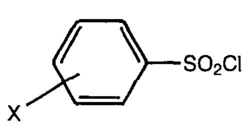

(I)

in der X für ein Halogenatom, eine Phenylgruppe, eine Phenoxygruppe oder eine niedere Alkylgruppe steht, bei dem eine Verbindung der allgemeinen Formel II

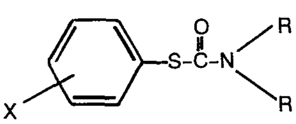

(II)

in der X die im Vorstehenden angegebene Bedeutung hat und die R's für die gleiche niedere Alkylgruppe stehen, in wäßrigem Medium mit Chlorgas umgesetzt wird.

Die Benzolsulfonylchloride (I) können als Zwischenprodukte zur Herstellung von bekannten, herbizid-aktiven Benzolsulfonylharnstoff-Derivaten verwendet werden.

EP 0 138 139 A2

NIHON TOKUSHU NOYAKU SEIZO K.K., Tokyo/Japan

Bi-Ma
IVa/ZP

# Neues Verfahren zur Herstellung substituierter Benzolsulfonylchloride

Die vorliegende Erfindung betrifft ein neues Verfahren zur Herstellung substituierter Benzolsulfonylchloride, das die verschiedenartigen Nachteile und Mängel der konventionellen Verfahren in der Industriepraxis überwindet und die vorgenannten Verbindungen in hoher Reinheit und mit hoher Ausbeute industriell vorteilhaft zu erzeugen vermag.

Im einzelnen betrifft die vorliegende Erfindung ein Verfahren zur Herstellung substituierter Benzolsulfonylchloride der nachstehenden Formel (I)

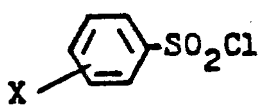

... (I) ,

in der X für ein Halogen-Atom, eine Phenyl-Gruppe, eine Phenoxy-Gruppe oder eine niedere Alkyl-Gruppe steht, bei dem eine Verbindung der nachstehenden Formel (II)

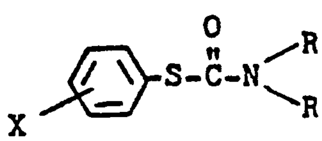

... (II)

in der X die im Vorstehenden angegebene Bedeutung hat und die R's für die gleiche niedere Alkyl-Gruppe stehen, in wäßrigem Medium mit Chlor-Gas umgesetzt wird.

Die substituierten Benzolsulfonylchloride der Formel (I), die die angestrebten Endprodukte des Verfahrens gemäß der vorliegenden Erfindung sind, sind bekannte Verbindungen, die industriell als Zwischenprodukte sehr wertvoll für die Herstellung substituierter Benzolsulfonylisocyanate sind, die als Rohstoffe für die Erzeugung herbizid aktiver substituierter Benzolsulfonylharnstoff-Derivate wie 1-(2-Chlorophenylsulfonyl)-3-(4-methoxy-6-methyl-1,3,5-triazin-2-yl)harnstoff (der in der JP-OS 122384/1977 beschriebenen Verbindung) und 1-(2-Biphenylylsulfonyl)-3-(4,6-dimethoxy-1,3,5-triazin-2-yl)harnstoff (der in der JP-OS 123168/1982 beschriebenen Verbindung) eingesetzt werden.

In der Vergangenheit war für die Erzeugung der substituierten Benzolsulfonylchloride der Formel (I) allgemein ein Verfahren verfügbar, das die Diazotierung eines substituierten Anilins und Umsetzung des Diazotierungsprodukts mit Schwefligsäure-Gas umfaßt, wie nachstehend schematisch dargestellt ist.

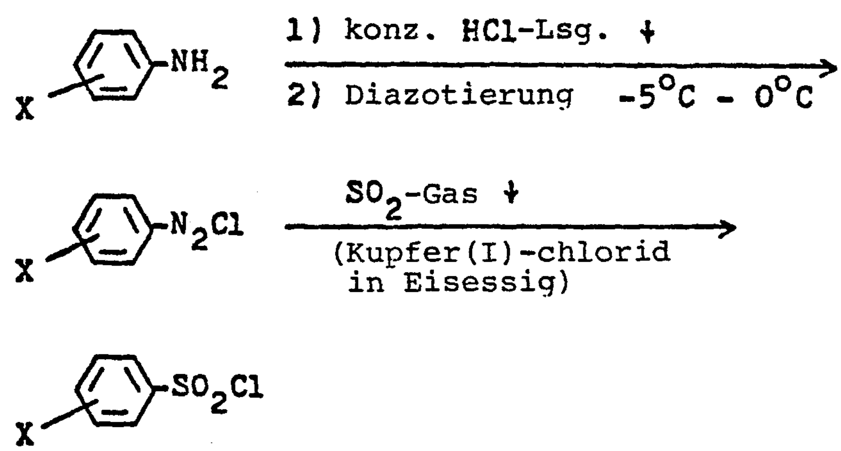


Hinsichtlich der praktischen Durchführung im industriellen Maßstab haften dem vorstehenden bekannten Verfahren viele Nachteile an. Erstens ist das als Ausgangsstoff einzusetzende substituierte Anilin teuer. Zweitens ist das als Zwischenprodukt entstehende Diazonium-Salz unbeständig. Drittens muß wegen der Verwendung eines Essigsäure-Lösungsmittelsystems das entstehende, Essigsäure enthaltende Abwasser aufbereitet werden. Der mit der Behandlung des sauren Abwassers verbundene Arbeitsgang der Rückgewinnung der Essigsäure ist industriell sehr schwierig und mit Nachteilen behaftet. Viertens ist der Einsatz eines großen Überschusses an Schwefligsäure-Gas erforderlich, und dies birgt die Gefahr von Umweltverschmutzungen in sich, so daß eine im industriellen Maßstab arbeitende Entschwefelungsanlage erforderlich ist. Infolgedessen ist das Verfahren industriell nicht zweckmäßig. Fünftens kann die Verwendung von Kupfer(I)-chlorid eine Wasserverschmutzung nach sich ziehen, und es it notwendig, einen Anlagenteil zur Behandlung des Kupfer(I)-chlorids vorzusehen. Somit wirft dieses herkömmliche Verfahren viele Probleme auf, die gelöst werden müssen.

Seitens der Anmelderin wurden ausgedehnte Untersuchungen zur Entwicklung eines Verfahrens zur Herstellung substituierter Benzolsulfonylchloride durchgeführt, das die genannten technischen Probleme zu lösen vermag. Im Zuge dieser Untersuchungen wurde unerwarteterweise gefunden, daß durch Umsetzung des S-Aryldialkylthiocarbamats der Formel (II) in wäßrigem Medium mit Chlor-Gas die verschiedenen technischen Probleme des herkömmlichen Verfahrens gelöst werden können und mittels einer sehr einfachen Arbeitsweise das gewünschte substituierte Benzolsulfonylchlorid der Formel (I) in hoher Reinheit mit hohen Ausbeuten hergestellt werden kann.

Die Untersuchungen seitens der Anmelderin haben gezeigt, daß mit Hilfe des Verfahrens gemäß der vorliegenden Erfindung die Reaktion einfach und sicher unter stationären Bedingungen ohne Verwendung eines organischen Reaktionslösungsmittels, das schwierig zurückzugewinnen ist, und eines Reaktionsmediums, das in seiner Handhabung gefährlich ist, durchgeführt werden kann und aufgrunddessen die substituierten Benzolsulfonylchloride der Formel (I) mit hoher Reinheit in hohen Ausbeuten mit einer guten Reproduzierbarkeit ihrer Qualität hergestellt werden können. Weiterhin wurde gefunden, daß das Verfahren gemäß der vorliegenden Erfindung wirtschaftlich vorteilhaft durchgeführt werden kann, wobei die verschiedenen Nachteile des herkömmlichen Verfahrens ausgeschaltet werden.

Die in dem Verfahren gemäß der vorliegenden Erfindung eingesetzte Ausgangs-Verbindung der Formel (II) kann leicht hergestellt werden, beispielsweise mittels des nachstehenden Verfahrens, das in Journal of Organic Chemistry, Band 31, 3980 (1966) und Band 33, 2249 (1968), beschrieben ist.

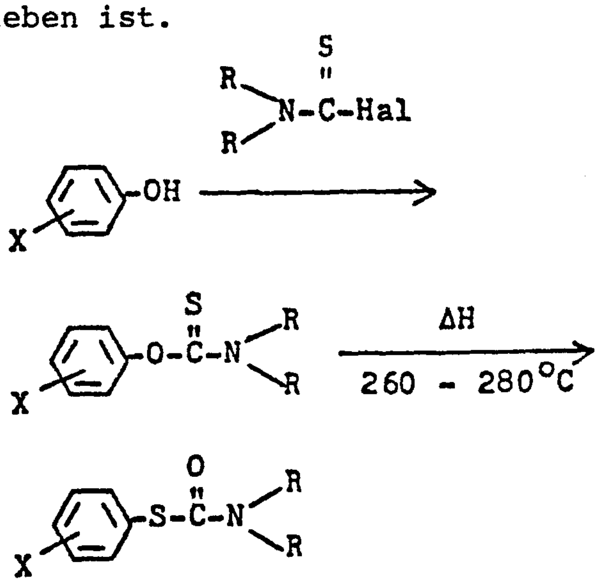


(II)

Das in dem vorstehenden Reaktionsschema als Ausgangsmaterial eingesetzte substituierte Phenol ist zu weitaus niedrigeren Kosten verfügbar als das in der oben erwähnten herkömmlichen Reaktion eingesetzte substituierte Anilin. Die vorstehende Reaktion kann mittels einer sehr einfachen gewöhnlichen Umsetzungstechnik durchgeführt werden, speziell in der Weise, daß zuerst eine Zwei-Phasen-Reaktion zur Anwendung gelangt und dann eine thermische Umlagerungsreaktion durchgeführt wird, und die Verbindung der Formel (II) in hoher Reinheit kann leicht mit hohen Ausbeuten erhalten werden. In der industriellen Praxis wird in der vorstehenden Reaktion anstelle der Zwei-Phasen-Reaktion eine Base wie 4-Dimethylaminopyridin eingesetzt, wodurch die Verbindung der Formel (II) in sehr hohen Ausbeuten gewonnen werden kann. Weiterhin kann die Base mittels einer einfachen Rückgewinnungstechnik wiedergewonnen und in vorteilhafter Weise im Kreislauf geführt werden.

Aus diesem Grunde ist es das Ziel der vorliegenden Erfindung, ein neues Verfahren zur Herstellung der substituierten Benzolsulfonylchloride der allgemeinen Formel (I) verfügbar zu machen, das für die Industriepraxis von Vorteil ist.

Der Reaktionsweg in dem Verfahren gemäß der vorliegenden Erfindung läßt sich folgendermaßen darstellen:

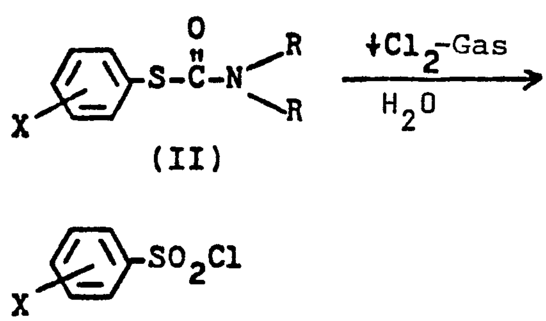

(II)

(I)

(Hierin haben X und R die oben angegebenen Bedeutungen).

In dem vorstehenden Reaktionsschema bezeichnet X ein Halogen-Atom wie Chlor, Fluor, Brom oder Iod, eine Phenyl-Gruppe, eine Phenoxy-Gruppe oder eine niedere Alkyl-Gruppe. Beispiele für diese Alkyl-Gruppe sind Methyl-, Ethyl-, Propyl-, Isopropyl- sowie n- (iso-, sec- oder tert-)Butyl-Gruppen.

Die zwei R's bezeichnen die gleichen niederen Alkyl-Gruppen, wie sie oben beispielhaft genannt sind. Vorzugsweise bezeichnen die beiden R's Methyl oder Ethyl.

Zu speziellen Beispielen für die Ausgangs-Verbindung der Formel (II) in dem durch das vorstehende Reaktionsschema veranschaulichten Verfahren gemäß der vorliegenden Erfindung zählen S-2-Chlorophenyldimethylthiocarbamat, S-2-Phenylphenyldimethylthiocarbamat, S-2-Phenoxyphenyldimethylthiocarbamat und S-2-Tolyldimethylthiocarbamat.

Das Verfahren der vorliegenden Erfindung wird anhand des folgenden typischen Beispiels im einzelnen näher beschrieben.

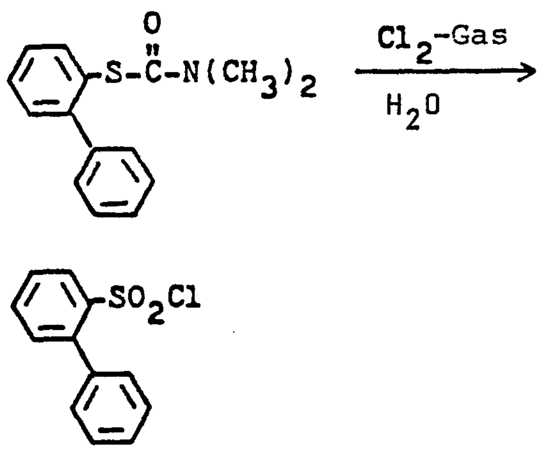

0138139

Das vorstehende Verfahren kann unter Verwendung sämtlicher inerter Lösungsmittel oder Verdünnungsmittel durchgeführt werden. Zu speziellen Beispielen für solche Lösungsmittel oder Verdünnungsmittel zählen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid und Sulfone und Sulfoxide wie Dimethylsulfoxid und Sulfolan.

Das Verfahren gemäß der vorliegenden Erfindung kann allgemein bei einer Temperatur zwischen etwa -20°C und dem Siedepunkt der Mischung, vorzugsweise zwischen etwa 0°C und etwa 30°C, durchgeführt werden. Die Reaktion wird zweckmäßigerweise unter normalem Atmosphärendruck durchgeführt, jedoch ist es auch möglich, unter erhöhtem oder vermindertem Druck zu arbeiten.

Die umzusetzende Menge Chlor-Gas kann passend gewählt werden, jedoch beträgt sie vorzugsweise wenigstens etwa 5 mol pro 1 mol der Verbindung der Formel (II).

Das Verfahren zur Herstellung der Verbindung der Formel (II) in dem obigen Verfahren läßt sich beispielsweise durch das folgende Reaktionsschema erläutern:

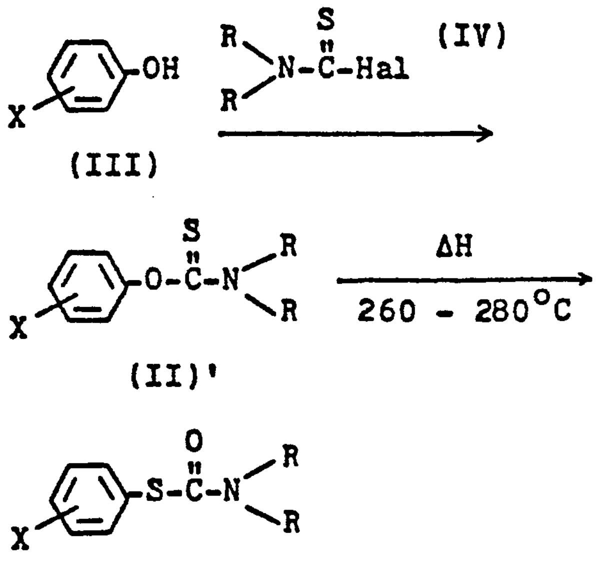


(III)

(II)'

(II)

(In den Formeln bezeichnen X und R die gleichen Substituenten wie im Vorstehenden).

In dem obigen Reaktionsschema sind spezielle Beispiele für die Ausgangs-Verbindung der Formel (III) 2-Chlorophenol, 2-Phenylphenol, 2-Phenoxyphenol und 2-Methylphenol.

Spezielle Beispiele für die Verbindung der Formel (IV) sind N,N-Dimethylthiocarbamoylchlorid und N,N-Diethylthiocarbamoylchlorid.

Das oben genannte Verfahren zur Herstellung der Verbindungen der Formel (II) kann leicht nach der in der bekannten Literaturstelle, die im Vorstehenden angeführt ist, angegebenen Arbeitsweise durchgeführt werden.

Die folgenden Beispiele erläutern die vorliegende Erfindung im einzelnen. Es ist jedoch darauf hinzuweisen, daß die Erfindung nicht auf diese Beispiele allein beschränkt ist.

Beispiel 1-a

17 g 2-Phenylphenol, 14,8 g N,N-Dimethylthiocarbamoylchlorid und als Phasentransfer-Katalysator 1,14 g Triethylbenzylammoniumchlorid wurden in 100 ml Toluol gelöst, und unter Kühlung auf eine Temperatur unterhalb der Raumtemperatur wurden 50 g einer 20-proz. wäßrigen Natriumhydroxid-Lösung tropfenweise zugegeben. Die Reaktion wurde bei der gleichen Temperatur 2 h durchgeführt. Die organische Schicht wurde von der Reaktionsmischung abgetrennt und mit Wasser gewaschen. Dann wurde das Toluol abgedampft, wonach O-2-Phenylphenyldimethylthiocarbamat in einer Reinheit von mehr als 95 % mit einer Ausbeute von mehr als 90 % erhalten wurde. Anschließend wurde das Produkt bei 260°C bis 280°C 15 min einer thermischen Umlagerungsreaktion unterworfen, wonach 23,1 g S-2-Phenylphenyldimethylthiocarbamat der nachstehenden Formel mit einem Umwandlungsgrad von 95 % erhalten wurden.

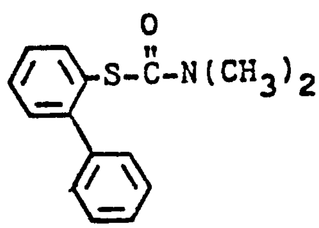

Beispiel 1-b

Das in Beispiel 1-a erhaltene S-2-Phenylphenyldimethylthiocarbamat (23,1 g) wurde in 50 ml einer 70-proz. wäßrigen Essigsäure-Lösung gelöst, und unter Eiskühlung wurde Chlor-Gas in einer Menge von 5 mol auf 1 mol des Thiocarbamats eingeleitet, wonach 21,2 g des gewünschten 2-Phenylbenzolsulfonylchlorids der nachstehenden

Nit 169

Formel (Netto-Ausbeute: 84 %) erhalten wurden. Schmelzpunkt: 93-97°C.

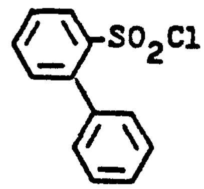

(Verbindung Nr. 1)

Beispiel 2

17 g 2-Phenylphenol, 12,4 g N,N-Dimethylthiocarbamoylchlorid, 12,2 g 4-Dimethylaminopyridin und 20,2 g Triethylamin wurden in 100 ml Toluol gelöst, und die Lösung wurde 4 h auf 70°C erhitzt. Das in der organischen Schicht ausgefallene Salz wurde durch Waschen mit Wasser entfernt. Weiterhin wurde die organische Schicht mit Wasser gewaschen, und das Toluol wurde abgedampft, wonach O-2-Phenylphenyldimethylthiocarbamat in einer Reinheit von mehr als 95 % mit einer Ausbeute von mehr als 93 % erhalten wurde. Anschließend wurde das Produkt in gleicher Weise wie in Beispiel 1-a einer thermischen Umlagerungsreaktion mit einem Umwandlungsgrad von 95 % unterworfen, und 25 ml Toluol und 50 ml Wasser wurden hinzugefügt. Unter Eiskühlung wurde Chlor-Gas in einer Menge von 5 mol auf 1 mol des Thiocarbamats eingeleitet. Die wäßrige Schicht wurde abgetrennt, und die organische Schicht wurde mit Wasser gewaschen. Nach dem Abdampfen des Toluols wurden 23,0 g 2-Phenylbenzolsulfonylchlorid, der gleichen Verbindung, die auch in Beispiel 1-b hergestellt wurde, (Netto-Ausbeute: 91 %) erhalten. Schmelzpunkt: 93-97°C.

 0138139

Beispiel 3

14,5 g 2-Chlorophenol, 12,4 g N,N-Dimethylthiocarbamoylchlorid, 12,2 g 4-Dimethylaminopyridin und 20,2 g Triethylamin wurden in 100 ml Toluol gelöst. Die Lösung wurde in gleicher Weise wie in Beispiel 2 zur Reaktion gebracht, wonach 18,2 g (Netto-Ausbeute: 86 %) 2-Chlorobenzolsulfonylchlorid der nachstehenden Formel erhalten wurden. Siedepunkt: 114-115°C/2 mbar (1,5 mmHg).

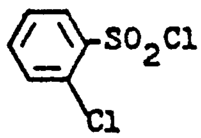

(Verbindung Nr. 2)

Mittels des gleichen Verfahrens wie in den Beispielen 1-a und 1-b wurden die nachstehenden Verbindungen synthetisiert.

Verbindung Nr. 3:

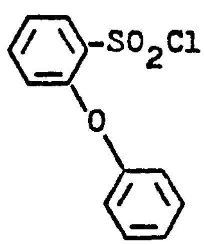

(Netto-Ausbeute: 84 %)

Verbindung Nr. 4:

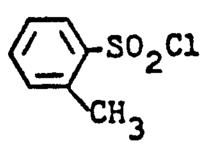

(Netto-Ausbeute: 85 %)

## Beispiel 4

Das Verfahren gemäß der vorliegenden Erfindung wurde im industriellen Maßstab nach der gleichen Arbeitsweise wie in Beispiel 2 durchgeführt. Die Ergebnisse sind in Tabelle 1 dargestellt.

### Tabelle 1

| Ausgangs-gangsstoffe | Produkt | Reaktions-Lösungsmittel und Base | Reaktions-Temperatur u.-Zeit | Ausbeute % | Reinheit % | Netto-Ausbeute % | Eigenschaft |
|---|---|---|---|---|---|---|---|
| 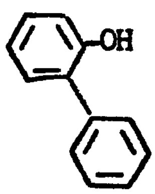<br>1,7 kg<br>(10 mol)<br><br>$(CH_3)_2N\overset{S}{\overset{\|}{C}}-Cl$<br>1,24 kg<br>(10 mol)<br><br>$Cl_2$-Gas<br>(entsprechend<br>50 mol) | 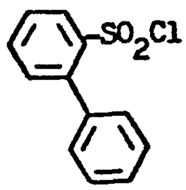<br>2,30 kg | 4-DMAP<br>1,22 kg<br>(10 mol)<br><br>TEA<br>2,02 kg<br>(20 mol)<br><br>Toluol<br>12 l<br><br>Wasser<br>5 l | wie in Beispiel 2 | 98,5 | 95,0 | 92,0 | Schmp. 93-97°C |

Anmerkungen:

(1) 4-DMAP: 4-Dimethylaminopyridin

(2) TEA: Triethylamin

## Patentansprüche

1. Verfahren zur Herstellung eines substituierten Benzolsulfonylchlorids der nachstehenden Formel (I)

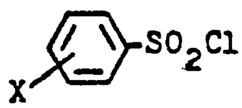

... (I) ,

in der X für ein Halogen-Atom, eine Phenyl-Gruppe, eine Phenoxy-Gruppe oder eine niedere Alkyl-Gruppe steht, dadurch gekennzeichnet, daß eine Verbindung der nachstehenden Formel (II)

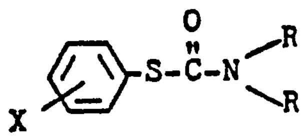

... (II) ,

in der X die im Vorstehenden angegebene Bedeutung hat und die R's für die gleiche niedere Alkyl-Gruppe stehen, in wäßrigem Medium mit Chlor-Gas umgesetzt wird.

2. Verfahren zur Herstellung eines substituierten Benzolsulfonylchlorids der nachstehenden Formel (I),

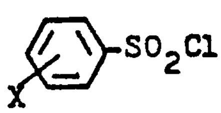

... (I) ,

in der X für ein Halogen-Atom, eine Phenyl-Gruppe, eine Phenoxy-Gruppe oder eine niedere Alkyl-Gruppe steht, dadurch gekennzeichnet, daß ein substituiertes Phenol der nachstehenden Formel (III)

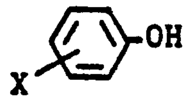

... (III)

,

in der X die im Vorstehenden angegebene Bedeutung hat, mit einer Verbindung der nachstehenden Formel (IV)

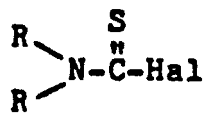

... (IV)

,

in der die R's für die gleiche niedere Alkyl-Gruppe stehen und Hal für ein Halogen-Atom steht, zu einer Verbindung der nachstehenden Formel (II)'

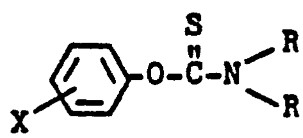

... (II)'

,

in der X und R die im Vorstehenden angegebenen Bedeutungen haben, umgesetzt wird, die entstandene Verbindung einer thermischen Umlagerung in die Verbindung der nachstehenden Formel (II)

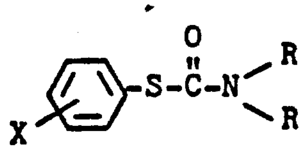

... (II)

,

in der X und R die im Vorstehenden angegebenen Bedeutungen haben, unterworfen wird und die enstandene Verbindung in wäßrigem Medium mit Chlor-Gas umgesetzt wird.

3. Verfahren gemäß Anspruch 1, dadurch gekennzeichnet, daß man bei Temperaturen zwischen 0°C und 30°C arbeitet.

4. Verfahren gemäß Anspruch 1, dadurch gekennzeichnet, daß man auf 1 Mol der Verbindung der Formel (II) wenigstens 5 Mol Chlor einsetzt.

5. Verfahren gemäß Anspruch 1, dadurch gekennzeichnet, daß man als Ausgangsverbindung der Formel (II) O-2-Phenyl-phenyldimethylthiolcarbamat oder O-2-Phenoxy-phenyl-dimethylthiolcarbamat einsetzt.